Europäisches Patentamt

European Patent Office

Office européen des brevets

(19)

(11) EP 1 417 968 A2

(12) **EUROPEAN PATENT APPLICATION**

(43) Date of publication:
**12.05.2004 Bulletin 2004/20**

(51) Int Cl.[7]: **A61K 35/64**, A61K 31/05,
A61P 35/00

(21) Application number: **03025195.3**

(22) Date of filing: **04.11.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **06.11.2002 IT mi20022352**

(71) Applicant: **Universita Degli Studi di Catania
95125 Catania (IT)**

(72) Inventors:
  • **Renis, Marcella
    95125 Catania (IT)**
  • **Scifo, Christian
    95125 Catania (IT)**

  • **Cardile, Venera
    95125 Catania (IT)**
  • **Russo, Alessandra
    95125 Catania (IT)**
  • **Falsaperla, Mario
    95125 Catania (IT)**
  • **Consoli, Rosanna
    95125 Catania (IT)**
  • **Capasso, Francesco
    95125 Catania (IT)**
  • **Vanella, Angelo
    95125 Catania (IT)**

(74) Representative: **Minoja, Fabrizio, Dr.
Bianchetti Bracco Minoja S.r.l.
Via Rossini, 8
20122 Milano (IT)**

(54) **Antitumour compositions containing propolis and resveratrol**

(57)    The present invention relates to combinations of resveratrol and propolis, and optionally vinorelbine, for tumour therapy, in particular for the treatment of prostatic carcinoma.

EP 1 417 968 A2

## Description

### FIELD OF THE INVENTION

The present invention relates to combinations of resveratrol and propolis, and optionally vinorelbine, for tumour therapy.

### BACKGROUND OF THE INVENTION

The antiproliferative and chemopreventive properties of propolis, a well-known apiculture product, and of its components, in particular caffeic acid and galangine derivatives, have been under study for some time (J. Ethnopharmacol. 80 (2002) 67-73; Cancer Research 54 (1994) 1865-1870; ibidem 55 (1995) 3576-3593; ibidem 57 (1997) 440-446; Mutation Research, 448 (2001) 135-150).

The antiproliferative and chemopreventive activity of resveratrol (3',4',5'-trihydroxystilbene), a natural compound found in grape (*Vitis vinifera*) skin and in red wine is also well known and investigated (Science, 275 (1997) 218-220; Bioinorg. Med. Chem. Letters 8 (1998) 3187-3192; Mutation Research, 496 (2001), 171-180).

Vinorelbine is a vinca alkaloid effective in the treatment of solid tumours, in particular breast tumour, androgen-resistant prostatic carcinoma and non-small-cells lung carcinoma. A semisynthetic derivative known under the trade name Navelbine® (vinorelbine ditartrate) is used in human therapy. Vinorelbine exerts antitumour activity by reversibly binding tubulines, causing dissolution of the mitotic spindle and metaphase arrest. Like other compounds acting on microtubules, vinorelbine induces apoptosis through activation of the bc1-2-associated protein kinases and the tumour suppressing gene TP53. Vinorelbine is generally administrated in combination with 5-fluorouracil. Even though some typical side effects (such as nausea, vomit and hair loss) of vinca alkaloids are reduced, vinorelbine still causes myelodepression and neuropathy, further to multi-drug resistance. This latter phenomenon is responsible for a remarkable reduction in the effectiveness of vinorelbine therapy and increases the treatment period. As the side effects are directly proportional to the administered dose, it would be useful to reduce the dose without jeopardising the effectiveness.

### DISCLOSURE OF THE INVENTION

It has now been found that combinations of resveratrol and propolis exert a synergistic action in respect of cytotoxicity (*LDH release*), DNA fragmentation (TMOM as measured by *comet assay*) and cell viability (MTT) in tumour cells, in particular in prostatic carcinoma cells.

It has also been found that resveratrol and propolis, alone or in combination with one another, enhance vinorelbine's cytotoxic effects (*LDH release*), thus allowing to reduce the dose in tumour therapy, in particular in the treatment of estrogen-dipendent tumours, more particularly prostate tumours.

In a first embodiment, the invention relates to pharmaceutical compositions containing propolis and resveratrol as the active ingredients, in admixture with suitable excipients.

According to a further embodiment, the invention relates to a combined preparation containing vinorelbine and at least one compound selected from resveratrol and propolis for simultaneous, separated or sequential use in tumour therapy.

The combined preparations according to the invention are preferably used in the therapy of estrogen-dependent tumours, more preferably prostatic tumours.

Finally, the invention relates to the combined use of resveratrol and propolis, for the preparation of an antitumour agent and to the use of resveratrol and propolis, alone or in combination with one another, for the preparation of an adjuvant agent having sinergistic activity with vinorelbine.

### DETAILED DISCLOSURE OF THE INVENTION

The compositions of the invention containing propolis and resveratrol are preferably in the form of tablets, capsules, solutions or suspensions. The dosage in humans will depend on more factors, such as weight, conditions, sex and age of the patients and will be determined by those skilled in the art of medicine based on pharmacokinetics, tossicological and pharmacodynamic characteristics of the compositions. For oral administration, the doses range from 200 to 400 mg/die of resveratrol and from 400 to 800 mg/die of propolis.

The combined preparations (kits) of the invention may contain a vinorelbine conventional administration form and separate administration forms, among those indicated above, containing a combination of resveratrol and propolis or only one of the two components.

Vinorelbine can be administered simultaneously, previously or subsequently to either resveratrol or propolis, or both.

Resveratrol and propolis activity was evidenced in androgen-resistant prostate-tumour DU145 cells. When treated with vinorelbine and resveratrol, the cells undergo more marked necrosis than when treated with resveratrol only. Likewise, treatment with vinorelbine and propolis causes more marked apoptosis compared with than propolis only. Treatment with propolis in combination with resveratrol causes synergistic cytotoxic effects comparable to those obtainable with vinorelbine only.

The use of resveratrol and propolis, alone or in combination with one another, allows therefore to reduce the dosage of conventional chemotherapeutics, in particular vinorelbine, thus increasing their therapeutic index, or their effectiveness even against tumour-resistant cell lines.

The invention is described in greater detail in the following example.

EXAMPLE

Androgen-resistant prostate tumour DU145 cells have been used. This cell line, originated from a cerebral metastasis of a prostatic tumour, represents the last step of the tumour progression, against which traditional chemotherapeutics, such as vinorelbine, are almost ineffective.

DV 146 cells were seeded in 35 mm dishes with EMEM containing 10% fetal calf serum, penicillin (50 IU/ml) /streptomycin (50 µg/ml), 1 mM glutamine and 1% of non-essential amino acids and incubated at 37°C in a mixture $CO_2$: air 5 : 95. After 24 hours, the plates were treated for 48 hours as follows:

- 2.5 µM, 5 µM, 10 µM, 30 µM vinorelbine ditartrate (Navelbine®);
- 100 and 200 µM resveratrol ethanol solution (dried resveratrol, Sigma Aldrich);
- propolis extract ethanol solution (50 and 100 µM/ml).

Propolis dried extract (ethanol extract) was purchased by Carlo Sessa S.p.A. (Italy) and titred for CAPE (caffeic acid phenyl ethyl ester) and flavonoids, both expressed as galangine. Their content was 10.44% and 9.04% respectively.

At the end of the treatment the cells were harvested and analysed for cytotoxicity measured as plasmatic membrane deaggregation (LDH release), percentage of metabolically active viable cells (MTT assay) and genomic DNA integrity (COMET assay). The results are reported in the table.

Table

| TREATMENTS | MIT | LDH release | TMOM |
|---|---|---|---|
| CONTROL | 100% | 6% ± 3 | 60 ± 40.2 |
| 2.5 µM VINORELBINE | 30% ± 4 | 22% ± 6 | 229 ± 84.5 |
| 5 µM VINORELBINE | 29% ± 5 | 20% ± 4 | 230 ± 71.9 |
| 10 µM VINORELBINE | 38% ± 5 | 21% ± 4 | --- |
| 30 µM VINORELBINE | 35% ± 5 | 22% ± 3 | 130 ± 69.3 |
| 100 µM RESVERATROL | 53% ± 4 | 12% ± 3 | --- |
| 200 µM RESVERATROL | 32% ± 3 | 48% ± 5 | 190 ± 100 |
| PROPOLIS 50 µg/ml | ---- | 11% ± 3 | --- |
| 100 µg/ml PROPOLIS | 62% ± 5 | 25% ± 4 | 350 ±138.2 |
| 5 µM VINORELBINE + 200 µM RESVERATROL | 38% ± 3 | 75% ± 5 | 160 ± 68.3 |
| 5 µM VINORELBINE + 100 µg/ml PROPOLIS | 62% ± 3 | 40% ± 4 | 620 ± 93.2 |
| 200 µM RESVERATROL + 100 µg/ml PROPOLIS | **51% ± 2.5\*** | 86% ± 5 | 588 ± 88.5 |
| 200 µM RESVERATROL + 100 µM/ml PROPOLIS + 2.5 µM VINORELBINE | **43% ± 3\*** | 84% ± 5 | 685 ± 95.4 |

$$TMOM = TDNA \times TD.$$

with:

TDNA - % DNA present in the comet tail;
TD = length of the comet tail.

**Vinorelbine,** in the concentration range 2.5-30 μM, significantly reduces without dose-dependent effects the viability (MTT assay) compared with the values observed for the untreated controls (cell viability of the controls: 100%; cell viability after treatment with vinorelbine: 29-38%).

The cytotoxic effect caused by vinorelbine, evaluated as the percentage of lactic dehydrogenase release (LDH release) amounts to 19-22% at 2.5 to 30 μM, the LDH release in the controls being 6%.

After treatment with 5 μM vinorelbine, genomic DNA, analyzed by COMET assay, showed a significant fragmentation (TMOM: 230) compared with untreated controls (TMOM: 60). This phenomenon could be, according to literature data, of the apoptotic type.

The viability of the cells treated with **resveratrol** (MTT assay) was reduced in a dose-dependent manner: 100 μM = 53%; 200 μM = 32%; the latter value being comparable to that obtained with vinorelbine. The cytotoxic effect (LDH release), was also dose-dependent (100 μM: 12% released LDH, 200 μM: 48% released LDH) and, at high doses, more marked than that observed with 5 μM vinorelbine (22% released LDH). At a concentration of 200 μM, moreover, a more marked DNA fragmentation than that induced by high doses of vinorelbine was observed, (200 μM resveratrol TMOM: 190; 30 μM vinorelbine: 130).

The **propolis** extract induced evident alterations on cell morphology in a dose-dependent manner (50 and 100 μg/ml) and reduced the viability (MTT assay) to 62% viable cells, thus resulting less effective than vinorelbine and resveratrol in respect of this parameter. LDH release percentage significantly increased compared with the untreated controls only at high dosage (100 μg/ml: 25%), which gave the same results as vinorelbine. Rather marked DNA fragmentation with a TMOM typical of the apoptotic injury was observed after treatment with propolis 100 μg/ml (TMOM: 350).

As far as the association **vinorelbine (5 μM) + resveratrol (200 μM)** is concerned, the reduction of cell viability was the same as that observed after treatment with a high vinorelbine dosage (30 μM: 38%). Instead, the percentage of released LDH, increased up to 75%. As regards viability, synergistic effects were not observed with the combination **vinorelbine (5 μM) + propolis (100 μg/ml)**, compared with those observed after treatment with the micronutrient only (62%). This treatment seems to be only moderately cytotoxic, in view of the limited increase in LDH release (40% released LDH) compared with the treatment with vinorelbine only. On the contrary, an increase in DNA injury was observed in the COMET assay.

The combination of resveratrol (200 μM) and propolis (100 μg/ml) determined a synergistic increase in LDH release and DNA injury, similarly to the triple combination of resveratrol (200 μM), propolis (100 μg/ml) and vinorelbine (5 μM), which induced an even more marked injury on genomic DNA.

**Claims**

1. Pharmaceutical compositions containing as the active ingredients propolis and resveratrol in admixture with suitable excipients.

2. Compositions as claimed in claim 1 in the form of tablets, capsules, solutions or suspensions.

3. Combined preparation containing vinorelbine and at least one compound selected from resveratrol and propolis for simultaneous, separate or sequential use.

4. Preparation as claimed in claim 3 containing vinorelbine and resveratrol.

5. Preparation as claimed in claim 3 containing vinorelbine and propolis.

6. Use of resveratrol and propolis for the preparation of an agent for the treatment of tumours.

7. Use as claimed in claim 6, wherein the tumours are estrogen-dependent tumours.

8.  Use as claimed in claim 7, wherein the tumour is prostatic carcinoma.

9.  Use of resveratrol for the preparation of an adjuvant agent for vinorelbine tumour therapy.

10. Use of propolis for the preparation of an adjuvant agent for vinorelbine tumour therapy.